Europäisches Patentamt

European Patent Office

Office européen des brevets

(19)

(11) Veröffentlichungsnummer : **0 003 305**
**B2**

(12) **NEUE EUROPÄISCHE PATENTSCHRIFT**

(45) Veröffentlichungstag der neuen Patentschrift :
26.03.86

(21) Anmeldenummer : **79100107.6**

(22) Anmeldetag : **15.01.79**

(51) Int. Cl.⁴ : **C 07 C 11/09**, C 07 C   7/148,
C 07 C 41/36

(54) Verfahren zur Gewinnung von Isobuten aus Isobuten enthaltenden C4-Kohlenwasserstoffgemischen.

(30) Priorität : **19.01.78 DE 2802198**

(43) Veröffentlichungstag der Anmeldung :
**08.08.79 Patentblatt 79/16**

(45) Bekanntmachung des Hinweises auf die Patenterteilung : **24.03.82 Patentblatt 82/12**

(45) Bekanntmachung des Hinweises auf die Entscheidung über den Einspruch : **26.03.86 Patentblatt 86/13**

(84) Benannte Vertragsstaaten :
**BE CH DE FR GB IT NL SE**

(56) Entgegenhaltungen :
**DE-A- 2 011 826
DE-B- 1 216 865
DE-B- 1 934 422
US-A- 3 170 000
US-A- 3 634 535
Hydrocarbons from Petroleum, 1953, Seiten 86-87
Azeotropic Data-III, Advances in Chemistry, Serie s 116, 1973
Journal of Catalysis 46, 1977, Seiten 49-57
Die Akte enthält technische Angaben, die nach dem Eingang der Anmeldung eingereicht wurden und die nicht in dieser Patentschrift enthalten sind.**

(73) Patentinhaber : **BASF Aktiengesellschaft
Carl-Bosch-Strasse 38
D-6700 Ludwigshafen (DE)**

(72) Erfinder : **Brunner, Erwin, Dr.
Neuhausener Weg 1
D-6700 Ludwigshafen 29 (DE)**
Erfinder : **Schubert, Eckart, Dr.
Bruesseler Ring 47
D-6700 Ludwigshafen (DE)**
Erfinder : **Lindner, Alfred, Dr.
Ringstrasse 22
D-6712 Bobenheim-Roxheim 1 (DE)**
Erfinder : **Merger, Franz, Dr.
Max-Slevogt-Strasse 25
D-6710 Frankenthal (DE)**
Erfinder : **Volkamer, Klaus, Dr.
Heidelberger Ring 21
D-6710 Frankenthal (DE)**
Erfinder : **Strohmeyer, Max, Dr.
Woogstrasse 41
D-6703 Limburgerhof (DE)**
Erfinder : **Sandrock, Gerhard, Dr.
Albrecht-Duerer-Ring 36c
D-6710 Frankenthal (DE)**

**Beschreibung**

Die vorliegende Erfindung betrifft ein Verfahren zur Gewinnung von Isobuten aus Isobuten enthaftenden C$_4$-Kohlenwasserstoffgemischen durch Umsetzung des Gemisches mit Isobutanol, Abtrennung des gebildeten tertiären Äthers und Zerlegung desselben bei erhöhten Temperaturen.

Es ist bereits bekannt, Isobuten durch Anwendung von Schwefelsäure-Extraktionsverfahren aus C$_4$-Kohlenwasserstoffgemischen zu gewinnen. Bei diesen Schwefelsäure-Extraktionsverfahren muß Schwefelsäure hoher Konzentration verwendet werden, und infolgedessen ist die Verwendung kostspieliger Materialien für die Ausrüstung erforderlich. Da weiterhin Nebenreaktionen des Isobutens, beispielsweise Dimerisation, Polymerisation, Hydration und dergleichen während der Extraktion erfolgen, ist das Schwefelsäure Extraktionsverfahren hinsichtlich Ausbeute und Qualität der Produkte nicht stets zufriedenstellend.

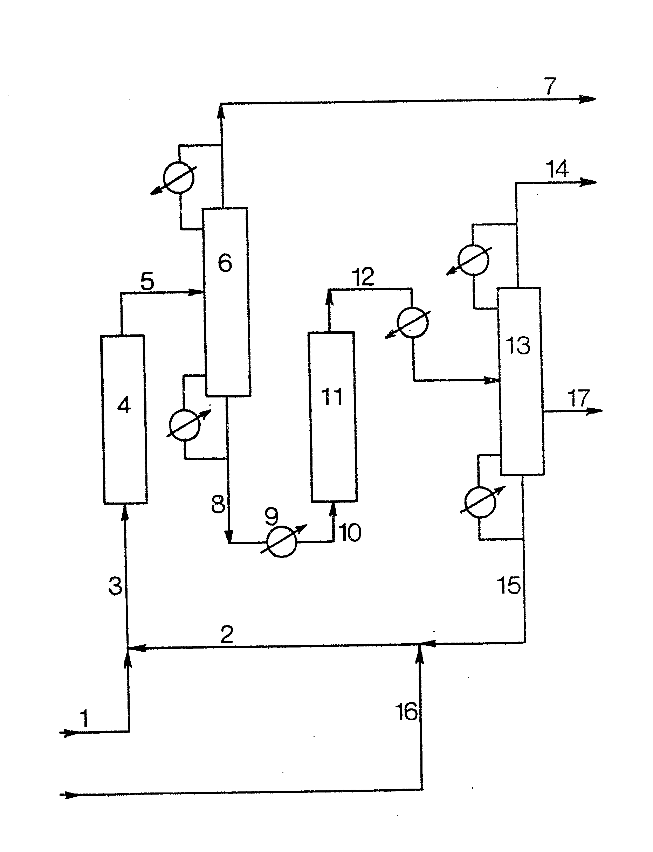

Es ist weiter aus der DE-AS-12 16 865, besonders Beispiel 20, oder DE-AS-19 34 422 ein Verfahren zur Gewinnung von Isobuten aus Isobuten enthaltenden C$_4$-Kohlenwasserstoffgemischen bekannt, bei dem das Gemisch mit Methanol in Gegenwart eines in einem Festbett angeordneten Ionenaustauscher-Katalysators umgesetzt wird, das bei der Verätherung erhaltene Reaktionsgemisch destilliert wird, wobei man als Kopfprodukt die nicht umgesetzten C$_4$-Kohlenwasserstoffe und als Bodenprodukt den gebildeten Methyl-tert.-butyläther und Methanol abzieht, das Bodenprodukt in einer zweiten, einen sauren Katalysator enthaltenden Reaktionszone bei erhöhter Temperatur in Isobuten und Methanol zerlegt wird und das Reaktionsgemisch der Zerlegung in einer Destillationskolonne in ein Isobuten-Kopfprodukt und ein das Methanol enthaltendes Bodenprodukt aufgetrennt wird. Die bekannten Verfahren haben jedoch den Nachteil, daß das erhaltene Isobuten-Produkt aufgrund von Azeotropbildung etwa 2 % Methanol und als weitere Verunreinigung Dimethyläther enthält, so daß es für viele Verwendungszwecke weiteren aufwendigen Reinigungsoperationen unterworfen werden muß. So sind gemäß dem Verfahren der DE-AS-19 34 422 (vgl. besonders Beispiel 9 in Verbindung mit Figur 4), die folgenden zusätzlichen Verfahrensschritte erforderlich :

1. Destillation des Isobuten-Produktes zur Dimethyläther-Abtrennung

2. Anschließende Extraktion des Isobutens mit Wasser zur Entfernung des Methanols

3. Destillation des aus der Extraktion erhaltenen Methanol-Wasser-Gemisches zur Rückgewinnung des Methanols

Außerdem muß das nach der extraktiven Behandlung mit Wasser erhaltene wasserhaltige Isobuten für eine Reihe von Verwendungszwecken noch einer Trocknung unterzogen werden.

Aber nicht nur bei der Abtrennung des Isobuten-Produktes aus dem Reaktionsprodukt der Zerlegungsstufe, sondern auch bei der Herstellung des Methyl-tert.-butyläthers in der Verätherungsstufe wird bei der Destillation des Reaktionsproduktes der Verätherung zur Abtrennung der nicht umgesetzten Kohlenwasserstoffe aufgrund von Kohlenwasserstoff/Methanol-Azeotropen ein Methanol enthaltendes Kopfprodukt erhalten. Daher muß auch in der Verätherungsstufe eine aufwendige Wasserwäsche des die nicht umgesetzten Kohlenwasserstoffe enthaltenden Kopfproduktes eingeschaltet werden, um die Methanol-Verluste des Verfahrens niedrig zu halten und ein Methanol-freies Gemisch der nicht umgesetzten Kohlenwasserstoffe zu erhalten, wie es bei der Weiterverarbeitung dieses Gemisches in vielen Fällen erforderlich ist (vgl. z. B. DE-AS-19 34 422, insbesondere Beispiel 9 in Verbindung mit Figur 4).

Neben der Verwendung von Methanol und allenfalls Äthanol als Alkohole für die Verätherungsreaktion sind zwar bereits allgemein primäre Alkohole als mögliche Reaktionspartner für die Umsetzung zum tertiären Äther genannt worden (vgl. z. B. die bereits vorstehend genannten DE-PS-12 16 865 oder DE-AS-19 34 422).

Gegen die Verwendung höherer primärer Alkohole z. B. von C$_3$-oder C$_4$-Alkoholen spricht, daß solche höheren primären Alkohole unter den Reaktionsbedingungen der Zerlegungsstufe in Gegenwart eines sauren Katalysators leicht zu Olefinen dehydratisiert werden können. So wird z. B. in der bereits genannten DE-AS-19 34 422, Spalte 3, 1. Absatz ausdrücklich darauf hingewiesen, Methanol, das sich nicht dehydratisieren läßt, als Alkohol anzuwenden, um die unerwünschte Bildung von Olefinen in der Zerlegungsstufe zu vermeiden.

Wegen der vorstehend beschriebenen Nachteile haben die bekannten Verfahren zur Isobutengewinnung unter Zerlegung des in einer ersten Verätherungsstufe erhaltenen tertiären Äthers keine großtechnische Anwendung gefunden, sondern sind nur papierner Stand der Technik geblieben, und man war daher für die großtechnische Gewinnung von Isobuten auf die Anwendung von Schwefelsäure-Extraktionsverfahren mit den diesen Verfahren anhaftenden Mängeln und Nachteilen angewiesen.

Der Erfindung lag daher die Aufgabe zugrunde, ein Verfahren zur Gewinnung von Isobuten aus Isobuten enthaltenden C$_4$-Kohlenwasserstoffen zur Verfügung zu stellen, welches die Nachteile der bekannten Verfahren nicht aufweist.

Es wurde nun ein vorteilhaftes Verfahren gefunden zur Gewinnung von Isobuten aus Isobuten enthaltenden C$_4$-Kohlenwasserstoffgemischen durch Umsetzung des Gemisches mit einem primären Alkohol in Gegenwart eines Ionenaustauschers in der Wasserstofform, wobei dieser in einem Festbett angeordnet ist, Destillation des bei der Verätherung erhaltenen Reaktionsgemisches, wobei man als

2

Kopfprodukt das $C_4$-Kohlenwasserstoff-Raffinat und als Bodenprodukt den gebildeten Alkyl-tert.-butyläther, gegebenenfalls noch mit überschüssigem primärem Alkohol abzieht, Zerlegung des Bodenproduktes in einer zweiten, einen sauren Katalysator enthaltenden Reaktionszone bei erhöhter Temperatur in Isobuten und primären Alkohol, Destillation des Gemisches aus Isobuten und primärem Alkohol, wobei als Kopfprodukt Isobuten und als Bodenprodukt der primäre Alkohol abgezogen werden, und Rückführung des erhaltenen primären Alkohols in die Reaktionszone für die Verätherung, welches dadurch gekennzeichnet ist, daß man als primären Alkohol Isobutanol verwendet.

Nach dem neuen Verfahren läßt sich aus dem nach der Verätherungsstufe erhaltenen Reaktionsgemisch ein praktisch Alkohol-freies $C_4$-Kohlenwasserstoff-Raffinat durch einfache Destillation ohne Einschaltung einer Wasserwäsche abtrennen, da nicht umgesetztes Isobutanol überraschenderweise keine Azeotrope mit den $C_4$-Kohlenwasserstoffen bildet. Im allgemeinen beträgt die Konzentration an Isobutanol im $C_4$-Kohlenwasserstoff-Raffinat höchstens 50 Gew.-ppm, vorzugsweise höchstens 20 Gew.-ppm, insbesondere höchstens 5 Gew.-ppm. Für das erfindungsgemäße Verfahren ist daher ein wesentlich geringerer Trennaufwand erforderlich als bei den bekannten Verfahren. Ein weiterer Vorteil des erfindungsgemäßen Verfahrens besteht darin, daß in einfacher Weise gleichzeitig ein Isobuten hoher Reinheit und ein weitgehend isobutenfreies $C_4$-Kohlenwasserstoff-Raffinat erhalten werden können. Ein solches weitgehend isobutenfreies $C_4$-Kohlenwasserstoff-Raffinat wird z. B. für bestimmte Verwendungszwecke, wie die Herstellung von sek.-Butanol, Methyläthylketon, Buten-1, Octenen oder Maleinsäureanhydrid benötigt.

Es ist weiter ein Vorteil des neuen Verfahrens, daß als $C_4$-Kohlenwasserstoffgemisch unmittelbar butadienhaltige $C_4$-Fraktionen, wie sie z. B. aus Äthylenanlagen oder Butan-/Buten-Dehydrier-Anlagen erhalten werden, verwendet werden können. Eine vorherige Butadien-Extraktion aus der $C_4$-Fraktion ist nicht erforderlich.

Überraschenderweise wird nach dem erfindungsgemäßen Verfahren Isobuten in hoher Ausbeute erhalten. Dies war unerwartet, da in der US-PS 3 170 000, Spalte 3, beschrieben ist, daß bei der Verwendung von $C_3$- oder $C_4$-Alkoholen nur sehr schlechte Ausbeuten an tertiärem Äther erhalten werden. Auch aus der US-PS 3 634 535, besonders Spalte 6, ist bekannt, daß die Umsetzung von Isobuten mit Propanol nur in einer Ausbeute von etwa 50 % zur Bildung des tertiären Äthers führt, während bei der entsprechenden Umsetzung von Isobuten und Methanol Ausbeuten von etwa 90 bis 95 % erhalten werden.

Für das erfindungsgemäße Verfahren geeignete isobuten enthaltende $C_4$-Kohlenwasserstoffgemische werden beispielsweise beim thermischen oder katalytischen Cracken von Erdölprodukten, bei der Herstellung von Äthylen durch Pyrolyse von verflüssigtem Petroleumgas (LPG), Leichtbenzin (Naphtha), Gasöl oder dergleichen oder bei der katalytischen Dehydrierung von n-Butan und/oder n-Buten erhalten. Diese $C_4$-Kohlenwasserstoffgemische enthalten in der Regel neben dem Isobuten olefinische und paraffinische $C_4$-Kohlenwasserstoffe und können darüber hinaus noch Butadien, z. B. in einer Menge von bis zu 70 Gewichtsprozent, und höhere Actylene, wie Butin-1 und Butenin enthalten. Butadien enthaltende $C_4$-Kohlenwasserstoffgemische können als solche oder nach vorheriger Abtrennung des Butadiens aus dem $C_4$-Kohlenwasserstoffgemisch, z. B. durch Butadien-Extraktion unter Verwendung eines selektiven Lösungsmittels, verwendet werden. Die $C_4$-Kohlenwasserstoffgemische können außerdem noch $C_3$-Kohlenwasserstoffe wie Propan, Propen, Propin z. B. bis zu 10 Gewichtsprozent, enthalten. Die $C_4$-Kohlenwasserstoffgemische weisen im allgemeinen einen Isobutengehalt von 5 bis 95 Gewichtsprozent, vorzugsweise 10 bis 90 Gewichtsprozent, insbssondere 20 bis 70 Gewichtsprozent, auf. Vorzugsweise werden solche $C_4$-Kohlenwasserstoffgemische verwendet, die neben Isobuten n-Butan, Isobutan, Buten-1, trans-Buten-2 und cis-Buten-2 und gegebenenfalls Butadien-1,3 enthalten.

Das erfindungsgemäß zu verwendende Isobutanol wird z. B. als technisches Produkt üblicher Reinheit, beispielsweise mit einer Reinheit von mindestens 95 %, vorzugsweise mindestens 98 %, verwendet.

Als saure Kondensationsmittel für die Verätherung, die die erste Stufe darstellt, kommen Ionenaustauscher in der Wasserstoffform in Betracht. Geeignete Ionenaustauscher sind beispielsweise sulfonierte Kohlen, sulfonierte Phenol-Formaldehyd-Harze, sulfonierte von Cumaron-Inden-Kondensationsprodukten abgeleitete Harze sowie insbesondere sulfonierte Polystyrol-Harze wie Kern-sulfonierte vernetzte Styrol-Divinylbenzol-Copolymerisate, beträgt deren Menge im allgemeinen 0,01 bis 1 l Schütt-·volumen pro l Reaktorvolumen. Für die Verätherung werden Festbettreaktoren eingesetzt.

Für die Verätherung wird das $C_4$-Kohlenwasserstoffgemisch mit Isobutanol in Gegenwart des Ionenaustauschers in der Wasserstoffform im allgemeinen bei Temperaturen von 20 bis 120 °C, vorzugsweise 20 bis 100 °C, umgesetzt. Zweckmäßig werden Austrittstemperaturen des Reaktionsgemisches aus der Reaktionszone von 20 bis 70 °C, vorzugsweise 20 bis 60 °C, insbesondere 20 bis 50 °C, angewendet.

Die erfindungsgemäße Verätherung kann bei Normaldruck erfolgen. Es ist jedoch zweckmäßig, einen geringen Überdruck, z. B. Drücke von 1,01 bis 30 bar, insbesondere 2 bis 20 bar, insbesondere 2 bis 20 bar, anzuwenden. Das Isobuten enthaltende $C_4$-Kohlenwasserstoffgemisch kann dabei je nach Druck und Temperatur für die Umsetzung flüssig oder gasförmig eingesetzt werden. Vorzugsweise werden flüssige Isobuten enthaltende $C_4$-Kohlenwasserstoffgemische eingesetzt. Die Verätherung kann diskontinuierlich durchgeführt werden. Die Reaktionszeiten liegen bei diskontinuierlicher Arbeitsweise im allgemeinen zwischen 1 Minute und 5 Stunden. Vorzugsweise wird die Verätherung jedoch kontinuierlich durchgeführt, wobei das Verhältnis aus Reaktorvolumen in 1 und dem Durchsatz in l/h im allgemeinen 0,01 bis 5 Stunden, vorzugsweise 0,3 bis 1 Stunde beträgt.

Das Gewichtsverhältnis von Isobutanol zu dem im $C_4$-Kohlenwasserstoffgemisch enthaltenen Isobuten beträgt bei der Verätherung im allgemeinen 100 : 1 bis 1 : 1, vorzugsweise 20 : 1 bis 1,2 : 1, insbesondere 4 : 1 bis 1,3 : 1.

Das nach der Verätherung erhaltene Reaktionsgemisch, welches in der Regel noch im Überschuß zur Verätherung zugesetztes Isobutanol enthält, wird durch Destillation ohne Einschaltung einer Wasserwäsche aufgetrennt, wobei als Kopfprodukt ein weitgehend isobutenfreies $C_4$-Kohlenwasserstoffgemisch-Raffinat erhalten wird, welches im allgemeinen einen Isobuten-Gehalt von höchstens 5 Gewichtsprozent, vorzugsweise höchstens 2,5 Gewichtsprozent, insbesondere 1,5 Gewichtsprozent, aufweist.

Als Bodenprodukt der Destillation des nach der Verätherung erhaltenen Reaktionsgemisches wird der gegebenenfalls noch überschüssiges Isobutanol enthaltende tertiäre Äther erhalten.

Der erhaltene tertiäre Äther wird anschließend in der zweiten Stufe des Verfahrens in Gegenwart eines sauren Katalysators bei erhöhten Temperaturen in Isobuten und Isobutanol zerlegt. Als Ausgangsprodukt für die Zerlegung kann tertiärer Äther, der praktisch frei von Isobutanol ist, verwendet werden, der beispielsweise durch Verwendung einer Menge an Isobutanol bei der Verätherung, die höchstens der stöchiometrisch erforderlichen Alkoholmenge entspricht, oder durch Abtrennung, z. B. durch Abdestillieren, von überschüssig zugesetztem Isobutanol aus dem nach der Destillation des Reaktionsgemisches der Verätherung erhaltenen Bodenprodukt erhalten worden ist. Vorzugsweise wird der nach der destillativen Abtrennung des $C_4$-Kohlenwasserstoffgemisch-Raffinats als Bodenprodukt erhaltene tertiäre Äther ohne weitere Abtrennung von gegebenenfalls vorhandenem überschüssigem Isobutanol für die Zerlegung eingesetzt. Es ist jedoch auch möglich, nur einen Teil des überschüssigen Isobutanols abzutrennen.

Für die Zerlegung wird der tertiäre Äther verdampft und mit dem sauren Katalysator in der Dampfphase kontaktiert. Als saure Katalysatoren kommen beispielsweise Ionenaustauscher in der Wasserstofform, wie sulfonierte Kohlen, sulfonierte Phenol-Formaldehydharze, sulfonierte von Cumaron-Inden-Kondensationsprodukten abgeleitete Harze sowie insbesondere sulfonierte Polystyrolharze wie Kern-sulfonierte, vernetzte Styrol-Divinylbenzol-Copolymerisate in Betracht.

Weiter sind feste Phosphorsäurekatalysatoren, die Mono-oder vorzugsweise Polyphosphorsäure auf einem festen Trägermaterial enthalten, vorteilhaft geeignet. Geeignete Trägermaterialien für die Phosphorsäurekatalysatoren sind z. B. Aluminiumoxid, Kieselsäure, Aktivkohle, Kieselgur oder Bims. Vorzugsweise wird Kieselgel als Trägermaterial verwendet.

Weitere geeignete saure Katalysatoren sind saure Metallsulfate wie Natriumhydrogensulfat, Calciumhydrogensulfat, Aluminiumsulfate, Nickelsulfat, Kupfersulfat, Kobaltsulfat, Cadmiumsulfat, Strontiumsulfat. Diese sauren Metallsulfate können als solche verwendet werden. Vorzugsweise werden sie auf einem Trägermaterial angewendet. Geeignete Trägermaterialien sind z. B. Kieselgel, Aktivkohle, Aluminiumoxid oder Bims.

Weiter kommen Kieselgel oder Aluminiumoxid alleine als Katalysatoren für die Zerlegung in Betracht.

In einer weiteren Ausführungsform des erfindungsgemäßen Verfahrens wird für die Zerlegung als saurer Katalysator ein Metallphosphat, insbesondere ein Metallhydrogenphosphat, verwendet. Diese Phosphate können Phosphorsäure auch im Überschuß, die über die stöchiometrische Zusammensetzung der sauren Metallphosphate hinausgeht, enthalten, z. B. in einem Überschuß bis zu 65 %, vorzugsweise bis zu 20 %, insbesondere bis zu 10 %. Als derartige Metallphosphate können beispielsweise Magnesiumphosphate, Calciumphosphate, Strontiumphosphate, Bariumphosphate, Manganphosphate, Nickelphosphate, Kupferphosphate, Kobaltphosphate, Cadmiumphosphate, Eisen(II)-phosphate, Chromphosphate und insbesondere Aluminiumphosphate verwendet werden. Der Metall-Katalysator kann als solcher oder auf einem Trägermaterial verwendet werden. Geeignete Trägermaterialien sind beispielsweise Aluminiumoxid, Kieselsäure, Aktivkohle, Zinkoxid.

Die Menge des sauren Katalysators beträgt im allgemeinen etwa, 0,01 bis 1 kg, vorzugsweise etwa 0,03 bis 0,3 kg je 1 kg/h Durchsatz des tertiären Äthers durch den Reaktor. Vorzugsweise werden für die Zerlegung des tertiären Äthers Festbettreaktoren verwendet.

Die Zerlegungstemperatur des tertiären Äthers varüert in Abhängigkeit von der Art dessauter Katalysators und der Kontakzeit, liegt jedoch im allgemeinen bei Temperaturen von 50 bis 350 °C, vorzugsweise 80 bis 300 °C, insbesondere 100 bis 250 °C. Bei der Verwendung von Metallphosphaten oder Phosphorsäurekatalysatoren als Zerlegungskatalysatoren werden im allgemeinen Temperaturen von 80 bis 350 °C, vorzugsweise 90 bis 260 °C, insbesondere 100 bis 250 °C, angewendet.

Die Kontaktzeit des verdampften tertiären Äthers beträgt zweckmäßig 0,1 bis 20 Sekunden, vorzugsweise 1 bis 10 Sekunden.

Die Zerlegung des tertiären Äthers kann bei Normaldruck erfolgen. Es ist jedoch auch möglich, Überdruck anzuwenden, z. B. Drücke bis zu 30 bar, vorzugsweise bis zu 20 bar, insbesondere Drücke von 1 bis 10 bar. Die Zerlegung kann jedoch auch bei vermindertem Druck durchgeführt werden.

Die Zerlegung des tertiären Äthers kann diskontinuierlich erfolgen. Vorzugsweise wird sie jedoch kontinuierlich durchgeführt.

Das bei der Zerlegung erhaltene Reaktionsgemisch, welches als Reaktionsprodukte Isobuten und Isobutanol enthält, wird in Isobuten und Isobutanol aufgetrennt. Diese Auftrennung erfolgt durch

4

Destillation, wobei in einfacher Weise ein hochreines Isobuten mit einer Reinheit von mehr als 99,8 % erhalten werden kann. Das aus der Auftrennung erhaltene Isobutanol wird im allgemeinen der Verätherungstufe wieder zugeführt.

Bei kontinuierlicher Arbeitsweise und Rückführung des nach der Zerlegung des tertiären Äthers und anschließender Aufarbeitung des Reaktionsgemisches erhaltenen Isobutanols in die Verätherungszone kann es bei dem neuen Verfahren zweckmäßig sein, aus dem Isobutanolstrom zur Entfernung von eventuell angereicherten Verunreinigungen einen Isobutanolteilstrom abzuzweigen. Zweckmäßig wird aus dem Isobutanolstrom ein Teilstrom abgezweigt, der 0,1 bis 10 Gewichtsprozent, vorzugsweise 0,5 bis 5 Gewichtsprozent des Isobutanolstromes beträgt. In einer vorteilhaften Ausführungsrom des Verfahrens wird der Isobutanolteilstrom in an sich bekannter Weise in Gegenwart eines Dehydratisierungskatalysators zu Isobuten dehydratisiert, wodurch die Ausbeute an Isobuten anders also bei den bekannten Verfahren zusätzlich erhöht wird.

Zweckmäßig wird die Dehydratisierung in der Gasphase an einem Katalysator durchgeführt. Geeignete Katalysatoren sind z. B. Kieselgel, Thoriumoxid, Titan (IV)-oxid und insbesondere Aluminiumoxid. Im allgemeinen werden für die Dehydratisierung Temperaturen von 250 bis 450 °C, vorzugsweise 300 bis 400 °C, angewendet.

In der Figur wird das erfindungsgemäße Verfahren schematisch erläutert. Das Isobuten enthaltende $C_4$-Kohlenwasserstoffgemisch (durch Leitung 1) und Isobutanol (durch Leitung 2) werden vermischt, und die erhaltene Mischung wird durch Leitung 3 dem Reaktor 4 für die Verätherung zugeführt, in dem sich der « saure » Ionenaustauscher, befindet. Der Reaktor ist als Festbettreaktor, z. B. als Strömungsrohr oder Schlaufenreaktor oder Kombination der beiden Reaktortypen ausgestaltet. Das erhaltene Reaktionsgemisch wird aus dem Reaktor über Leitung 5 abgezogen und einer ersten Destillationskolonne 6 zugeführt. Am Kopf der Destillationskolonne wird weitgehend isobutenfreies $C_4$-Kohlenwasserstoffgemisch (Raffinat) durch Leitung 7 abgezogen. Der als Bodenprodukt der Destillationskolonne 6 erhaltene tertiäre Äther, der gegebenenfalls noch im Überschuß zugesetztes Isobutanol enthält, wird über Leitung 8 zunächst dem Verdampfer 9 zugeführt und nach der Verdampfung über Leitung 10 in den Reaktor 11 eingeleitet, in dem sich der saure Katalysator befindet. Reaktor 11 ist im allgemeinen ein Fesbett-Reaktor. Das aus Reaktor 11 abgezogene Gemisch aus Isobuten und Isobutanol wird über Leitung 12 in die Destillationskolonne 13 geleitet, in der als Kopfprodukt hochreines Isobuten erhalten wird, welches über Leitung 14 abgezogen wird. Das als Bodenprodukt erhaltene Isobutanol wird über Leitung 15 und 2, gegebenenfalls nach ergänzender Zugabe von Isobutanol über Leitung 16, dem Reaktor 4 für die Verätherung wieder zugeführt. Über Leitung 17 wird zweckmäßig ein kleiner Isobutanol enthaltender Teilstrom zur Ausschleusung von eventuell gebildeten Verunreinigungen wie Diisobutyläther, Diisobuten, Triisobuten, abgezogen. Dieser Teilstrom kann einem Dehydratisierungsreaktor zugeführt werden, in dem zusätzlich Isobuten erhalten wird.

Nach dem erfindungsgemäßen Verfahren wird ein hochreines Isobuten erhalten, welches insbesondere für die Herstellung von hochmolekularen Polymeren des Isobutens geeignet ist.

Die folgenden Beispiele veranschaulichen die Erfindung.

## Beispiel 1

Die Verätherung wurde unter Verwendung eines $C_4$-Kohlenwasserstoffgemisches durchgeführt, welches den Rest (Raffinat) einer aus einer Äthylenanlage erhaltenen $C_4$-Fraktion darstellt, aus der das Butadien extrahiert worden war. Die Zusammensetzung des $C_4$-Kohlenwasserstoffgemisches nach der Butadien-Ektraktion war wie folgt :

| | |
|---|---|
| Isobutan | 1,9 Vol. % |
| n-Butan | 8,1 Vol. % |
| Isobuten | 46,0 Vol. % |
| Buten-1 | 26,7 Vol. % |
| trans-Buten-2 | 10,1 Vol. % |
| cis-Buten-2 | 7,0 Vol. % |
| Butadien-1,3 | 0,2 Vol. % |

Eine Mischung von 258 g je Stunde dieses $C_4$-Kohlenwasserstoffgemisches mit 320 ml je Stunde Isobutanol wurde in ein rohrförmiges Reaktionsgefäß aus rostfreiem Stahl eingeleitet, in dem sich 254 ml eines sulfonierten Polystyroldivinylbenzol-harzes in der Wasserstofform (Lewatit SPC 118, Kornfraktion 0,8 bis 1 mm) befanden. In dem Reaktionsgefäß wurde ein Druck von 12 bar aufrechterhalten. Das Reaktionsgemisch wurde mit einer Austrittstemperatur von 40 °C aus dem Reaktionsgefäß abgezogen und einer Destillationskolonne zugeführt, wobei am Kopf der Kolonne ein Buten-/Butan-Raffinat mit einem Isobutengehalt von weniger als 2 Gewichtsprozent erhalten wurde, das Raffinat war praktisch Isobutanol-frei und konnte daher ohne weitere Reinigungsoperationen, z. B. ohne Einschaltung einer Wasserwäsche, unmittelbar als Ausgangsstoff für weitere Reaktionen verwendet werden. Am Sumpf der Destillationskollone wurden 500 ml je Stunde Isobutyl-tert.-butyläther, der noch 24,3 Gewichtsprozent, bezogen auf das Gemisch, im Überschuß zugesetztes Isobutanol enthielt, abgezogen und in einen Verdampfer eingeleitet. Der verdampfte, auf 190 °C erhitzte Isobutyl-tert.-butyläther wurde zur Ätherspaltung in einen rohrförmigen Spaltreaktor, welcher als Spaltkatalysator saures Aluminiumphosphat (Molanteile 42 : 58) enthielt, eingeleitet und bei 190 °C in Isobuten und Isobutanol aufgespalten. Das Reaktionsprodukt der Ätherspaltung wurde in eine zweite Destillationskolonne eingeleitet, in der am Kopf 115 g je Stunde hochreines Isobuten der folgenden Zusammensetzung erhalten wurde :

| | | |
|---|---:|---|
| Isobuten | 99,85 | Gew.% |
| Isobutan | 730 | Gew.ppm |
| Butan | 3 | Gew.ppm |
| Buten-1 | 420 | Gew.ppm |
| trans-Buten-2 | 190 | Gew.ppm |
| cis-Buten-2 | 160 | Gew.ppm |
| Butadien-1,3 | 19 | Gew.ppm |

Die Ausbeute an Isobuten, bezogen auf das im eingesetzten $C_4$-Kohlenwasserstoffgemisch enthaltene Isobuten, betrug 97,7 %. Am Sumpf der zweiten Destillationskolonne wurden 320 ml je Stunde Isobutanol erhalten, welches wieder in die Verätherungsreaktion rückgeführt wurde.

Die Durchsätze an $C_4$-Kohlenwasserstoffgemisch und Isobutanol durch den Reaktor für die Verätherungsreaktion konnten um dem Faktor 4 erhöht werden bei praktisch unveränderten Reinheiten des bei der Destillation erhaltenen Buten-/Butan-Raffinates und des am Boden der Destillationskolonne erhaltenen, den Isobutyl-tert.-butyläther enthaltenden Produktes.


Vergleichsbeispiel

Die Verätherung wurde wie in Beispiel 1 beschrieben durchgeführt, wobei jedoch anstelle des Isobutanols die entsprechende stöchiometrische Menge Methanol eingesetzt wurde. Bei einem Durchsatz des Einsatzgemisches von 2 l/h je 1 l Reaktorvolumen betrug der Restgehalt an Isobuten in dem nach der Destillation erhaltenen Buten-/Butan-Raffinat noch mehr als 30 Gewichtsprozent. Das Buten-/Butan-Raffinat enthielt außerdem mehr als 1,5 Mol % Methanol, welches durch eine Wasserbehandlung aus dem Raffinat ausgewaschen wurde. Aus dem nach der Wasserwäsche erhaltemen Methanol-Wassergemisch wurde das Methanol zurückgewonnen und in die Verätherungsreaktion zurückgeführt.

Dagegen wird bei der Verwendung von Isobutanol (gemäß Beispiel 1) anstelle von Methanol durch einfache Destillation ein Buten-/Butan-Raffinat mit einem Isobutanol-Gehalt von weniger als 1 ppm erhalten.


Beispiel 2

Die Verätherung wurde unter Verwendung des $C_4$-Schnittes einer Äthylenanlage durchgeführt. Die Zusammensetzung des $C_4$-Kohlenwasserstoffgemisches war wie folgt :

| | |
|---|---:|
| Butan | 3,65 Gew.% |
| Isobutan | 1,41 Gew.% |
| Buten-1 | 20,44 Gew.% |
| Isobuten | 23,52 Gew.% |
| trans-Buten-2 | 4,95 Gew.% |
| cis-Buten-2 | 3,15 Gew.% |
| Butadien-1,3 | 42,31 Gew.% |
| Butadien-1,2 | 0,10 Gew.% |
| Butin-1 | 0,11 Gew.% |
| Butenin | 0,36 Gew.% |

Eine Mischung von 457 g je Stunde dieses Kohlenwasserstoffgemisches mit 320 ml je Stunde Isobutanol wurde wie in Beispiel 1 beschrieben umgesetzt. Der Isobutengehalt im nach der Destillation erhaltenen Buten-/Butan-Raffinat lag bei, 1,0 Gewichtsprozent.

Das Sumpfprodukt der ersten Destillation wurde verdampft und dann in einen rohrförmigen Spaltreaktor eingeleitet, in dem der Isobutyl-tert.-butyläther bei 190 °C in Isobuten und Isobutanol aufgespalten wurde. Das am Kopf der nachgeschalteten Destillationsstufe abgezogene Isobuten (107 g je Stunde) hatte die folgende Zusammensetzung :

| | |
|---|---:|
| Butan | 0,012 Gew.% |
| Isobutan | 0,041 Gew.% |
| Buten-1 | 0,042 Gew.% |
| Isobuten | 99,332 Gew.% |

| trans-Buten-2 | | 0,09 | Gew.% |
|---|---|---|---|
| cis-Buten-2 | | 0,11 | Gew.% |
| Butadien-1,3 | | 0,36 | Gew.% |
| Butadien-1,2 | | 0,007 | Gew.% |
| Butin-1 | | 0,003 2 | Gew.% |
| Butenin | | 0,002 8 | Gew.% |

Trotz eines Butadien-1,3-Gehaltes im Ausgangs-$C_4$-Kohlenwasserstoffgemisch von 42,31 Gewichtsprozent betrug der Butadien-1,3-Gehalt im Isobuten-Produkt lediglich 0,36 Gewichtsprozent. Ebenso wurden auch Butadien-1,2- sowie Butin-1 und Butenin sehr stark abgereichert.

Die Ausbeute an Isobuten, bezogen auf das im eingesetzten $C_4$-Kohlenwasserstoffgemisch enthaltene Isobuten betrug 96,5 %. Das am Sumpf der zweiten Destillationskolonne praktisch vollständig zurückgewonnene Isobutanol würde wieder in die Verätherungsreaktion zurückgeführt.

## Patentanspruch

Verfahren zur Gewinnung von Isobuten aus Isobuten enthaltenden $C_4$-Kohlenwasserstoffgemischen durch Umsetzung des Gemisches mit einem primären Alkohol in Gegenwart eines Ionenaustauschers in der Wasserstofform, wobei dieser in einem Festbett angeordnet ist, Destillation des bei der Verätherung erhaltenen Reaktionsgemisches, wobei man als Kopfprodukt das $C_4$-Kohlenwasserstoff-Raffinat und als Bodenprodukt den gebildeten Alkyl-tert.-butyläther, gegebenenfalls noch mit überschüssigem primären Alkohol abzieht, Zerlegung des Bodenproduktes in einer zweiten, einen sauren Katalysator enthaltenden Reaktionszone bei erhöhter Temperatur in Isobuten und primären Alkohol, Destillation des Gemisches aus Isobuten und primärem Alkohol, wobei als Kopfprodukt Isobuten und als Bodenprodukt der primäre Alkohol abgezogen werden, und Rückführung des erhaltenen primären Alkohols in die Reaktionszone für die Verätherung, dadurch gekennzeichnet, daß man als primären Alkohol Isobutanol verwendet.

## Claim

A process for obtaining isobutene from an isobutene-containing $C_4$-hydrocarbon mixture by reacting the mixture with a primary alcohol in the presence of an ion exchanger in its hydrogen form, the latter being arranged in a fixed bed, distilling the reaction mixture obtained in the etherification, the $C_4$-hydrocarbon raffinate being withdrawn as the overhead product, and the resulting alkyl-tert-butyl ether which may contain excess primary alcohol being withdrawn as the bottom product, separating the bottom product in a second reaction zone containing an acidic catalyst, at elevated temperature, into isobutene and primary alcohol, distilling the mixture of isobutene and primary alcohol, isobutene being withdrawn as the overhead product and the primary alcohol as the bottom product, and recycling the resulting primary alcohol to the reaction zone for etherification, characterized in that isobutanol is used as the primary alcohol.

## Revendication

Procédé pour l'extraction de l'isobutène de mélanges d'hydrocarbures en $C_4$, qui en contiennent, par réaction d'un mélange d'hydrocarbures en $C_4$ avec un alcool primaire en présence d'un échangeur d'ions dans sa forme $H^+$, disposé en un lit fixe, distillation du mélange réactionnel formé pendant l'éthérification, conduisant à un produit de tête composé des hydrocarbures en $C_4$ raffinés et à un résidu de distillation composé de l'éther alcoyltertiobutylique formé, ainsi que de l'alcool primaire éventuellement en excès, décomposition du résidu de distillation à température accrue, dans une deuxième zone réactionnelle, contenant un catalyseur acide, en isobutène et alcool primaire, distillation du mélange d'isobutène et alcool primaire, conduisant à la séparation de l'isobutène comme produit de tête, l'alcool primaire obtenu comme résidu de la distillation étant recyclé dans la zone d'éthérification, caractérisé en ce que l'alcool primaire utilisé est l'isobutanol.

7